# DEMANDE DE BREVET EUROPEEN

(11) **EP 2 138 151 A2**
(43) Date de publication de la demande: **30.12.2009**
(21) Numéro de dépôt: 09163906.2
(22) Date de dépôt: 26.06.2009
(51) Int. Cl.: A61K 8/03, A61Q 1/06

(54) **Composition cosmétique de maquillage et/ou de soin des lèvres**

(30) Priorité: 27.06.2008 FR 0854354
(71) Demandeur: L'ORÉAL, 75008 Paris (FR)
(72) Inventeur: Blin, Xavier, 75015, Paris (FR)
(74) Mandataire: Le Coupanec, Pascale A.M.P.

(57) **Abrégé**

La présente invention vise une composition cosmétique, notamment sous forme de stick, formée d'au moins un coeur liquide comprenant au moins une phase grasse liquide à température ambiante, et retenu, à l'intérieur d'une gaine solide comprenant au moins un corps gras solide à température ambiante.

Elle concerne également un procédé cosmétique de soin et/ou de maquillage des matières kératiniques consistant à appliquer sur ces dernières, au moyen d'un stick, une composition formée d'au moins un coeur liquide comprenant au moins une phase grasse liquide à température ambiante, et retenue, à l'intérieur d'une gaine solide.

## Description

La présente invention se rapporte à une composition cosmétique de maquillage et/ou de soin des lèvres.

La mise au point de formulations dédiées au maquillage et/ou au soin des lèvres, dotées de propriétés satisfaisantes en termes d'application, de confort, de tenue et de couvrance, mais également en terme d'effet maquillage, à savoir notamment l'obtention de brillance et de transparence, relève d'un objectif permanent.

Le stick à lèvres, apparu au début du siècle dernier, s'est aujourd'hui imposé et est reconnu par les utilisatrices comme le mode d'application plébiscité du maquillage de leurs lèvres. Ce mode d'application offre aux utilisatrices un moyen de choix, en termes de couvrance des lèvres et de diversité des couleurs. Toutefois, les utilisatrices, toujours à la recherche de nouvelles performances de la part de leur maquillage, souhaitent également que ce dernier brille et ceci de façon très importante.

On sait par ailleurs qu'il est possible de conférer un caractère brillant, classiquement par l'utilisation dans ce type de composition, d'huiles visqueuses à fort indice de réfraction, comme le malate de diisostéaryle et/ou des composés pâteux comme le polyacyl adipate de diglycéryle. Toutefois, l'usage de ces composés pose alors généralement des problèmes de collant et de migration. L'addition de charges peut certes suppléer à la migration mais malheureusement altère la brillance des compositions obtenues.

Pour l'ensemble de ces raisons, les formulations procurant généralement cet effet de brillance correspondent à des galéniques fluides, de type « gloss », encore plus particulièrement appelé « gloss liquide » ou « brillant à lèvres ».

Toutefois, de telles formulations ne sont généralement pas aptes à procurer conjointement les mêmes propriétés de confort d'application, de couvrance et de tenue que celles procurées par les formes galéniques « sticks » ou bâtons. En outre, ces galéniques souples ne peuvent pas être montées en stick sans perdre leurs propriétés de transparence et de brillance. En effet, la structuration en bâton ne permet pas de garder le caractère liquide ou semi-liquide et nécessite l'ajout de cires permettant cette structuration. Or, ces cires altèrent significativement la brillance et la transparence du mélange.

En conséquence, il demeure à ce jour un besoin de disposer d'une composition cosmétique de maquillage et/ou de soin des lèvres reproduisant les qualités de maquillage d'un stick en terme de facilité d'application, de couvrance et de tenue, et par ailleurs dotée des qualités de brillance et/ou de transparence intense des gloss.

La présente invention vise précisément à répondre à ce besoin.

Les inventeurs ont constaté qu'une formulation présentant une double architecture, à savoir une gaine solide de texture proche de celle d'un stick, entourant une formulation liquide de type gloss, était satisfaisante en ces termes.

Ainsi, l'objet de la présente invention concerne principalement une composition cosmétique, notamment sous forme de stick, formée d'au moins un coeur liquide comprenant au moins une phase grasse liquide à température ambiante, et retenu à l'intérieur d'une gaine solide comprenant au moins un corps gras solide à température ambiante.

Ainsi, l'invention concerne avantageusement des sticks ayant une structure du type coeur-gaine qui comprend deux compositions différentes disposées l'une par rapport à l'autre de façon à former un coeur entouré d'une gaine le long de leur axe vertical.

Ainsi, les structures considérées selon l'invention sont à distinguer de celles des capsules dans lesquelles une première composition enrobe l'intégralité d'une seconde composition qui forme le coeur de la capsule.

Selon l'invention, le terme « solide » caractérise l'état d'une composition à température ambiante et à pression atmosphérique (760 mm de Hg).

De façon préférée, la composition selon l'invention, lorsqu'elle est solide (partie correspondant à la gaine solide de la composition), est caractérisable par une valeur de dureté, pouvant être mesurée selon la méthode dite « du fil à couper le beurre », bien connue de l'homme du métier et mieux décrite ci-après dans le texte.

Au sens de la présente invention, on entend qualifier par « liquide » ou « fluide », par opposition à « solide », toute composition capable d'épouser la forme de son contenant, à température ambiante.

Plus précisément, la composition selon l'invention, lorsqu'elle est liquide (partie correspondant au coeur liquide de la composition), peut se présenter sous forme d'une pâte fluide pouvant notamment être caractérisée par une valeur de viscosité à température ambiante.

On entend par « température ambiante », la température de l'environnement dans lequel est disposée une composition et qui peut être fixée, pour des conditions expérimentales reproductibles, entre 20 et 25 °C.

Contre toute attente, les inventeurs ont réussi à mettre au point une architecture de composition cosmétique comprenant ces deux types de texture différents, chacune formant deux phases distinctes en contact l'une avec l'autre.

Plus précisément, le choix adéquat de la viscosité du coeur liquide, couplé à un dimensionnement approprié du tube central (diamètre) contenant la composition liquide relativement au diamètre de la gaine solide permet d'assurer un maintien, notamment par capillarité, du coeur liquide au centre de la gaine solide. Ainsi, par exemple, pour une composition liquide de viscosité à température ambiante d'environ 200 poises, un diamètre de la partie centrale du stick d'environ 2 mm, pour un diamètre extérieur du stick d'environ 12.7 mm, s'est avéré satisfaisant.

En outre, la gaine doit avoir une texture suffisamment solide et le coeur liquide une texture suffisamment liquide pour que l'un et l'autre ne se mélangent pas et conservent donc respectivement leurs propriétés intrinsèques.

Les inventeurs ont également constaté que pour que la gaine solide et le coeur ne se mélangent pas, le choix des phases grasses de la gaine solide et du coeur liquide peut s'avérer déterminant. En effet, les phases grasses de la gaine solide et du coeur liquide peuvent être avantageusement choisies de manière à ce qu'elles soient incompatibles, c'est-à-dire, qu'elles ne soient pas miscibles entre elles.

La non miscibilité de la gaine solide et du coeur liquide peut donc être le résultat d'une incompatibilité naturelle entre les différentes phases et notamment entre les différentes phases grasses.

Alternativement, on peut envisager qu'une composition selon l'invention comporte une ou plusieurs phases complémentaires localisées précisément entre le coeur liquide et la gaine solide pour garantir la non miscibilité du coeur liquide et de la gaine solide.

Une composition conforme à la présente invention présente l'avantage de concilier en une seule formulation les propriétés de brillance et de transparence d'une composition de type gloss et les propriétés de couvrance, couleurs et tenue du bâton de rouge à lèvres (ou stick).

De façon préférée, la présente invention vise une composition telle que définie précédemment, dans laquelle le coeur liquide est retenu à l'intérieur de la gaine solide, par capillarité.

Avantageusement, dans une composition selon la présente invention, les phases grasses de la gaine solide et du coeur liquide sont incompatibles ou non miscibles entre elles.

De manière préférée, la présente invention vise des compositions cosmétiques telles que définies précédemment, dont le coeur liquide comprend en outre des particules de silice pyrogénée.

Selon un mode de réalisation particulier, l'invention concerne une composition selon l'invention dans laquelle la gaine solide est structurée par au moins une cire.

Le coeur liquide, comme la gaine solide, conformes à l'invention comprennent chacun un milieu physiologiquement acceptable.

Par « milieu physiologiquement acceptable », on entend désigner un milieu convenant particulièrement à l'application d'une composition selon l'invention sur les lèvres.

Selon un mode de réalisation, les compositions conformes à l'invention peuvent être anhydres.

Par composition anhydre, on entend une composition comprenant moins de 10 % en poids, en particulier moins de 5 % en poids, en particulier moins de 3 % en poids, en particulier moins de 2 % en poids, et plus particulièrement moins de 1 % en poids d'eau par rapport au poids de la composition, figurée par le coeur liquide ou la gaine solide, selon le cas. Avantageusement la composition selon l'invention est anhydre.

Selon un autre aspect, la présente invention concerne un procédé cosmétique de soin et/ou de maquillage des matières kératiniques, et notamment des lèvres, consistant à appliquer sur ces dernières au moyen d'un stick une composition formée d'au moins un coeur liquide comprenant au moins une phase grasse liquide à température ambiante, et retenue, à l'intérieur d'une gaine solide comprenant au moins un corps gras solide à température ambiante.

La présente invention a encore pour objet un procédé cosmétique dans lequel la composition telle que définie précédemment est sous la forme d'un stick.

On entend par « matière kératinique » la peau, les ongles, les cheveux, les cils, les sourcils, les lèvres. Plus particulièrement, « matière kératinique » désigne les lèvres.

### COEUR LIQUIDE

Comme précisé précédemment, le coeur est fluide par opposition à la gaine l'enrobant qui est solide.

Cette fluidité peut être caractérisée en terme de viscosité.

Le coeur liquide selon la présente invention peut posséder une viscosité variant de 50 à 400 Poises et de préférence de 80 à 250 Poises. Cette viscosité est mesurée à l'aide d'un viscosimètre rotatif CONTRAVES TV^{®}, équipé d'un mobile « MS-r4 » à la fréquence de 60 Hz.

Le coeur liquide selon l'invention comporte au moins une phase grasse liquide, comprenant au moins un corps gras liquide à température ambiante et à pression atmosphérique.

### Phase grasse liquide

La phase grasse peut être continue. Dans ce cas, elle contient moins de 5 % d'eau, notamment moins de 1 % d'eau par rapport à son poids total et en particulier peut être sous forme anhydre.

La phase grasse de la formule du coeur liquide selon l'invention peut notamment comprendre, à titre de corps gras liquide, au moins une huile volatile ou non volatile ou leurs mélanges.

### Huiles volatiles

Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm Hg), en particulier allant de 1,3 Pa à 13 000 Pa (0,01 à 100 mm Hg), et plus particulièrement allant de 1,3 Pa à 1300 Pa (0,01 à 10 mm Hg).

Les huiles hydrocarbonées volatiles peuvent être choisies parmi les huiles hydrocarbonées ayant de 8 à 16 atomes de carbone, et notamment les alcanes ramifiés en C₈-C₁₆ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permethyls^{®}.

Comme huiles volatiles, on peut aussi utiliser les huiles de silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées telles que le nonafluorométhoxybutane ou le perfluorométhylcyclopentane, et leurs mélanges.

Il est également possible d'utiliser un mélange des huiles précédemment citées.

Une formule coeur liquide selon l'invention peut comprendre au moins une huile volatile en une teneur variant d'environ 0,1 % à environ 50 % en poids, en particulier variant d'environ 1 % à environ 40 % en poids, et plus particulièrement variant d'environ 2 % à environ 25 % en poids d'huile volatile par rapport au poids de la formule coeur liquide.

Avantageusement, le coeur liquide comprend moins de 5 % d'huile volatile en poids par rapport au poids total de la formule coeur liquide, voire n'en contient pas du tout.

### Huiles non volatiles

Au sens de la présente invention, on entend par « huile non-volatile », une huile ayant une pression de vapeur inférieure à 0,13 Pa et notamment des huiles de masse molaire élevée. Les huiles non volatiles peuvent être des huiles hydrocarbonées notamment d'origine végétale, des huiles d'origine synthétique ou minérale, des huiles siliconées, des huiles fluorées, ou leurs mélanges.

Comme huile hydrocarbonée non volatile, on peut notamment citer :
- les huiles apolaires hydrocarbonées ,
- les huiles apolaires de masse molaire élevée ayant, en particulier une masse molaire allant d'environ 400 à environ 10 000 g/mol, en particulier d'environ 650 à environ 10 000 g/mol, en particulier d'environ 750 à environ 7500 g/mol, et plus particulièrement variant d'environ 1000 à environ 5000 g/mol. Comme huile de masse molaire élevée utilisable dans la présente invention, on peut notamment citer les huiles choisies parmi :
   - les polymères lipophiles,
   - les huiles apolaires d'origine végétale,
   - et leurs mélanges,
- et leurs mélanges

Par exemple, une huile de masse molaire élevée peut être choisie parmi les polymères lipophiles tels que :
- les polybutylènes tels que L'INDOPOL H-100^{®} (de masse molaire MW=965 g/mol), L'INDOPOL H-300^{®} (MW=1340 g/mol), L'INDOPOL H-1500^{®} (MW=2160g/mol) commercialisés ou fabriqués par la société AMOCO,
- les polyisobutylènes, par exemple, hydrogénés tels que le PANALANE H-300 E^{®} commercialisés ou fabriqué par la société AMOCO (MW =1340 g/mol), le VISEAL 20000^{®} commercialisé ou fabriqué par la société SYNTEAL (MW=6000 g/mol), le REWOPAL PIB 1000^{®} commercialisé ou fabriqué par la société WITCO (MW=1000 g/mol), le PARLEAM^{®} de la société NOF,
- les polydécènes et les polydécènes hydrogénés tels que : le PURESYN 10^{®} (MW=723 g/mol), le PURESYN 150^{®} (MW=9200 g/mol) commercialisé ou fabriqués par la société MOBIL CHEMICALS, et
- des mélanges de ceux-ci.

Les huiles de silicone non volatiles utilisables selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme la phényl triméthicone, la phényl diméthicone, la diphényl diméthicone, les polydiméthylsiloxanes de viscosité inférieure ou égale à 100 cSt, et leurs mélanges.

A titre d'autres huiles polaires pouvant être mises en oeuvre selon l'invention, on peut également citer :
- les huiles polaires hydrocarbonées d'origine végétale telles que les esters de phytostéaryle, tels que l'oléate de phytostéaryle, l'isostéarate de physostéaryle et le glutamate de lauroyl/octyldodécyle/phytostéaryle (AJINOMOTO, ELDEW PS203), les triglycérides constitués d'esters d'acides gras et de glycérol, en particulier dont les acides gras peuvent avoir des longueurs de chaînes variant de C₄ à C₃₆, et notamment de C₁₈ à C₃₆, ces huiles pouvant être linéaires ou ramifiées, saturées ou insaturées ; ces huiles peuvent notamment être des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore des triglycérides d'acides caprylique/caprique comme ceux vendus par la société STEARINERIES DUBOIS ou ceux vendus sous les dénominations MIGLYOL 810^{®}, 812^{®} et 818^{®} par la société DYNAMIT NOBEL,
- les éthers de synthèse ayant de 10 à 40 atomes de carbone ;
- l'oligomère vinylpyrrolidone/1-hexadécène, ANTARON V-216 commercialisé ou fabriqué par la société ISP (MW=7300 g/mol)
- les esters de synthèse comme les huiles de formule R'₁COOR'₂, dans laquelle R'₁ représente un reste d'un acide gras linéaire ou ramifié comportant de 1 à 40 atomes de carbone et R'₂ représente une chaîne hydrocarbonée notamment ramifiée contenant de 1 à 40 atomes de carbone à condition que R'₁ + R'₂ soit ≥ 10. Les esters peuvent être notamment choisis parmi les esters, notamment d'alcool et d'acide gras comme par exemple :
   - l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en C₁₂ à C₁₅, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle ;
- les esters de polyols et les esters du pentaérythritol, comme le tétrahydroxystéarate/tétraisostéarate de dipentaérythritol,
- les acides gras supérieurs de C₁₂ à C₂₂ tels que l'acide oléique, l'acide linoléique, l'acide linolénique et leurs mélanges, et
- les carbonates de di-alkyle, les 2 chaînes alkyles pouvant être identiques ou différentes, tel que le dicaprylyl carbonate commercialisé sous la dénomination CETIOL CC^{®}, par COGNIS,
- les huiles polaires de masse molaire élevée ayant, en particulier une masse molaire allant d'environ 400 à environ 10 000 g/mol, en particulier d'environ 650 à environ 10 000 g/mol, en particulier d'environ 750 à environ 7500 g/mol, et plus particulièrement variant d'environ 1000 à environ 5000 g/mol.

Avantageusement, le coeur liquide d'une composition selon l'invention, comprend au moins un ester de polyol(s) et de dimère diacide d'acide gras, ou un de ses esters.

Comme huile polaire de masse molaire élevée utilisable dans la présente invention, on peut notamment citer les huiles choisies parmi :
- les esters d'acides gras linéaires ayant un nombre total de carbone allant de 35 à 70,
- les esters aromatiques,
- les esters d'alcools gras ou d'acides gras ramifiés en C₂₄-C₂₈,
- et leurs mélanges, - et leurs mélanges.

Par exemple, une huile polaire de masse molaire élevée peut être choisie parmi les esters tels que :
- les esters aromatiques tels que le tridécyle trimellitate (MW=757 g/mol) tel que le Liponate TDM^{®} de LIPO CHEMICALS,
- les esters d'alcools gras ou d'acides gras ramifiés en C₂₄-C₂₈ tels que ceux décrits dans la demande EP-A-0 955 039 et les esters du pentaérythritol, et notamment le citrate de triisoarachidyle (MW=1033,76 g/mol), le tétraisononanoate de pentaérythrityle (MW=697 g/mol), le triisostéarate de glycéryle (MW=891 g/mol), le tri décyl-2 tétradécanoate de glycéryle (MW=1143 g/mol), le tétraisostéarate de pentaérythrityle (MW=1202 g/mol), le tétraisostéarate de polyglycéryle-2 (MW=1232 g/mol) ou encore le tétra décyl-2 tétradécanoate de pentaérythrityle (MW=1538 g/mol),
- les esters et polyesters de dimère diol, tels que les esters de dimère diol et d'acide gras, et les esters de dimère diols et de diacide, tels que les Lusplan DD-DAS^{®} et Lusplan DD-DA7^{®} commercialisés par la société NIPPON FINE CHEMICAL et décrits dans la demande US 2004-175338, dont le contenu est incorporé dans la présente demande par référence.

Selon un mode de réalisation de l'invention, le coeur liquide de la composition selon la présente invention comprend au moins une huile non volatile, choisie parmi les polybutènes, les polyisobutylènes hydrogénés, les polydécènes, les polydécènes hydrogénés, les huiles de silicone, l'oligomère vinylpyrrolidone/1-hexadécène, l'isononanoate d'isonoyle, les esters de polyol(s) et de dimère diacide d'acide gras ou un de ses esters, les esters aromatiques tels que le tridécyle trimellitate, et leurs mélanges.

Les huiles non volatiles peuvent être présentes dans une formule coeur liquide selon l'invention en une teneur variant de 5 % à 99 % en poids, notamment de 15 % à 90 % en poids, et en particulier de 30 % à 80 % en poids, par rapport au poids total de la formule coeur liquide.

### Autres corps gras

D'autres corps gras peuvent être mis en oeuvre dans la formule coeur liquide selon l'invention, pour autant que leur nature et/ou leur teneur n'affecte pas les propriétés de viscosité et de brillance de la formule coeur liquide.

Ainsi, une formule liquide selon l'invention peut également comprendre au moins un corps gras choisi parmi les cires, les composés pâteux, les agents structurants et/ou épaississants, et leurs mélanges, tels que définis pour la phase grasse solide de la gaine solide, ci-après.

Toutefois, le coeur liquide contient généralement au moins deux fois moins de cires, composés pâteux, agents structurants et/ou épaississants que la gaine solide. Selon un mode de réalisation préféré, la teneur de ces composés dans une formule coeur liquide conforme à l'invention est inférieure à 15 % en poids, plus particulièrement inférieure à 10 % en poids, voire inférieure à 5 % en poids par rapport au poids total de la formule coeur liquide.

Avantageusement, le coeur liquide peut être exempt de cire et/ou de composé(s) pâteux et/ou de composés structurants et/ou épaississants.

Avantageusement, ladite phase grasse liquide peut contenir en outre des particules de silice pyrogénée.

### SILICE PYROGENEE

Les particules de silice pyrogénée convenant à la mise en oeuvre de l'invention peuvent être hydrophiles ou être traitées en surface afin d'être rendues hydrophobes.

Les silices pyrogénées hydrophiles peuvent être obtenues par hydrolyse à haute température d'un composé volatil du silicium dans une flamme oxhydrique, produisant une silice finement divisée. Les silices hydrophiles peuvent présenter un nombre important de groupements silanol à leur surface.

De telles silices hydrophiles sont par exemple commercialisées sous les dénominations « AEROSIL 130^{®} », « AEROSIL 200^{®} », « AEROSIL 255^{®} », « AEROSIL 300^{®} », « AEROSIL 380^{®} », « AEROSIL 972^{®} » par la société DEGUSSA, « CAB-O-SIL HS-5^{®} », « CAB-O-SIL EH-5^{®} », « CAB-O-SIL LM-130^{®} », « CAB-O-SIL MS-55^{®} », « CAB-O-SIL M-5^{®} » par la société CABOT.

Les silices pyrogénées hydrophobes peuvent être obtenues par modification de la surface de la silice par une réaction chimique générant une diminution du nombre de groupes silanols, ces groupes pouvant être notamment substitués par des groupements hydrophobes.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées « Silica silylate » selon le CTFA (6ème édition, 1995). Elles sont par exemple commercialisées sous les références « AEROSIL R812 » par la société DEGUSSA, « CAB-O-SIL TS-530^{®} » par la société CABOT.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (6ème édition, 1995). Elles sont par exemple commercialisées sous les références « AEROSIL R972^{®} », « AEROSIL R974^{®} » par la société DEGUSSA, « CAB-O-SIL TS-610^{®} », « CAB-O-SIL TS-720^{®} » par la société CABOT.

Des particules de silice pyrogénée peuvent être présentes dans la formule coeur liquide conforme à l'invention en une teneur variant d'environ 1 % à environ 30 % en poids, en particulier d'environ 2 à environ 20 % en poids, et plus particulièrement d'environ 5 à environ 15 % en poids par rapport au poids total de la formule coeur liquide.

La silice procure à la formule la contenant une texture épaisse, fondante et très brillante.

### GAINE SOLIDE

Comme précisé précédemment, lorsque la composition est à l'état solide et forme une gaine solide à température ambiante, elle peut être caractérisée en terme de dureté.

Plus précisément, comme indiqué précédemment, pour déterminer la dureté de la gaine solide, on utilise la méthode du « fil à couper le beurre » sur un échantillon de la gaine solide. Pour cela, un échantillon de la gaine solide est coulé à chaud dans un moule de sticks de 12,7 mm de diamètre. Le moule est ensuite refroidi au congélateur pendant une heure environ. Le stick de rouge à lèvres obtenu est ensuite conservé à 20 °C.

La dureté des échantillons est mesurée après 24 heures d'attente. La méthode utilisée consiste à couper transversalement le stick cylindrique de 12,7 mm de diamètre précédemment obtenu, à l'aide d'un fil rigide de diamètre 250 µm en tungstène, avançant à une vitesse de 100 mm/min.

La dureté des échantillons de la gaine solide selon l'invention correspond à la force maximale de cisaillement exercée par le fil sur le stick à 20 °C, exprimée en gramme, et est mesurée au moyen d'un dynamomètre DFGS2^{®}, commercialisé par la société INDELCO-CHATILLON.

Selon cette méthode la dureté de la gaine solide conforme à l'invention est comprise entre 30 et 500 g, en particulier entre 100 et 350 g, voire entre 100 et 300 g.

La gaine solide selon la présente invention a une épaisseur radiale comprise entre 2 et 10 mm et est de préférence comprise entre 3 mm et 9 mm.

La gaine solide entourant le coeur liquide est formée en tout ou partie d'une phase grasse comportant au moins un corps gras solide à température ambiante et à pression atmosphérique, pouvant être choisis parmi les composé pâteux, les cires, les agents structurants ou épaississants, et leurs mélanges.

### Composé pâteux

La gaine solide peut comprendre un corps gras solide tel qu'au moins un composé pâteux.

Par « pâteux » au sens de la présente invention, on entend un composé gras lipophile à changement d'état solide/liquide réversible, présentant à l'état solide une organisation cristalline anisotrope, et comportant à la température de 23 °C une fraction liquide et une fraction solide.

Le composé pâteux est de préférence choisi parmi les composés synthétiques et les composés d'origine végétale. Un composé pâteux peut être obtenu par synthèse à partir de produits de départ d'origine végétale. Le composé pâteux peut avantageusement choisi parmi :
- la lanoline et ses dérivés,
- les composés siliconés polymères ou non,
- les composés fluorés polymères ou non,
- les polymères vinyliques, notamment :
   - les homopolymères d'oléfines,
   - les copolymères d'oléfines,
   - les homopolymères et copolymères de diènes hydrogénés,
   - les oligomères linéaires ou ramifiés, homo ou copolymères de (méth)acrylates d'alkyles ayant de préférence un groupement alkyle en C₈-C₃₀,
   - les oligomères homo et copolymères d'esters vinyliques ayant des groupements alkyles en C₈-C₃₀, et
   - les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters,
- et leurs mélanges.

Parmi les esters, on préfère notamment :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649^{®} par la société SASOL,
- le propionate d'arachidyle commercialisé sous la marque Waxenol 801^{®} par ALZO,
- les esters de phytostérol,
- les triglycérides d'acides gras et leurs dérivés,
- les esters de pentaérythritol,
- les polyesters non réticulés résultant de la polycondensation entre un acide dicarboxylique ou un polyacide carboxylique linéaire ou ramifié en C₄-C₅₀ et un diol ou un polyol en C₂-C₅₀,
- les esters aliphatiques d'ester résultant de l'estérification d'un ester d'acide hydroxycarboxylique aliphatique par un acide carboxylique aliphatique,
- les polyesters résultant de l'estérification, par un acide polycarboxylique, d'un ester d'acide hydroxy carboxylique aliphatique, ledit ester comprenant au moins deux groupes hydroxyle tels que les produits Risocast DA-H ^{®}, et Risocast DA-L ^{®}
- les esters de dimère diol et dimère diacide, le cas échéant, estérifiés sur leur(s) fonction(s) alcool(s) ou acide(s) libre(s) par des radicaux acides ou alcools tels que le Plandool-G^{®},
- et leurs mélanges.

Parmi les composés pâteux d'origine végétale, on choisira de préférence un mélange de stérols de soja et de pentaérythritol oxyéthyléné (50E) oxypropyléné (5OP), commercialisé sous la référence^{®} par la société VEVY.

Le composé pâteux peut être présent dans la gaine solide en une teneur allant de 0,1 à 50 % en poids, notamment allant de 0,1 à 45 % en poids, et en particulier allant de 0,2 à 30 % en poids, par rapport au poids total de la gaine.

La gaine solide peut également comprendre une ou plusieurs cires. Avantageusement, elle en contient au moins une.

### Cires

Par « cire » au sens de la présente invention, on entend un composé gras lipophile, solide à température ambiante (25 °C), à changement d'état solide/liquide réversible, ayant une température de fusion supérieure ou égale à 30 °C pouvant aller jusqu'à 200 °C et notamment jusqu'à 120 °C.

En particulier, les cires convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :
Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées selon l'invention sont choisies parmi les cires, solides, à température ambiante d'origine animale, végétale, minérale ou de synthèse et leurs mélanges.

A titre illustratif des cires convenant à l'invention, on peut notamment citer les cires hydrocarbonées comme la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène telles que Performalène 500-L polyéthylène^{®} de NEW PHASE TECHNOLOGIES, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en C₈-C₃₂. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50^{®}, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S^{®} par la société HETERENE.

On peut encore citer les cires de silicone (C₃₀₋₄₅ alkyl diméthicone), les cires fluorées.

On peut également utiliser les cires obtenues par hydrogénation d'huile de ricin estérifiée avec l'alcool cétylique vendues sous les dénominations de Phytowax ricin 16L64^{®} et 22L73^{®} par la société SOPHIM. De telles cires sont décrites dans la demande FR-A- 2792190.

Comme cire, on peut utiliser un (hydroxystéaryloxy)stéarate d'alkyle en C₂₀-C₄₀ (le groupe alkyle comprenant de 20 à 40 atomes de carbone), seul ou en mélange.

Une telle cire est notamment vendue sous les dénominations « Kester Wax K 82 P^{®} », « Hydroxypolyester K 82 P^{®} » et « Kester Wax K 80 P^{®} » par la société KOSTER KEUNEN.

Comme microcires pouvant être utilisées selon l'invention, on peut citer notamment les microcires de carnauba telles que celle commercialisée sous la dénomination de MicroCare 350^{®} par la société MICRO POWDERS, les microcires de cire synthétique telles que celle commercialisée sous la dénomination de MicroEase 1145^{®} par la société MICRO POWDERS, les microcires constituées d'un mélange de cire de carnauba et de cire de polyéthylène telles que celles commercialisées sous les dénominations de Micro Care 300^{®} et 310^{®} par la société MICRO POWDERS, les microcires constituées d'un mélange de cire de carnauba et de cire synthétique telles que celle commercialisée sous la dénomination Micro Care 325^{®} par la société MICRO POWDERS, les micro cires de polyéthylène telles que celles commercialisées sous les dénominations de Micropoly 200^{®}, 220^{®}, 220L^{®} et 2505^{®} par la société MICRO POWDERS et les microcires de polytétrafluoroéthylène telles que celles commercialisées sous les dénominations de Microslip 519^{®} et 519 L^{®} par la société MICRO POWDERS.

La cire peut être présente dans la gaine solide selon l'invention en une teneur d'au moins 1 % en poids par rapport au poids total de la gaine solide, en particulier de 3 à 40 % en poids, et notamment de 5 à 30 %, tout particulièrement de 5 % à 25 %, voire 5 % à 20 % en poids par rapport au poids total de la gaine solide.

La gaine solide selon l'invention peut comprendre, outre les cires éventuellement présentes, au moins un agent structurant et/ou épaississant choisi parmi les polymères semi-cristallin, les gélifiants lipophiles et leurs mélanges.

### Polymères semi-cristallin

On entend par polymère semi cristallin des composés comportant au moins deux motifs, de préférence au moins 3 motifs et plus spécialement au moins 10 motifs de répétition. Par « polymère semi-cristallin », on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par « séquence », on entend généralement au moins 5 motifs de répétition identiques. La ou les séquences cristallisables sont alors de nature chimique différente de la ou des séquences amorphes.

Le polymère semi-cristallin a une température de fusion supérieure ou égale à 30 °C (notamment allant de 30 °C à 80 °C), de préférence allant de 30 °C à 60 °C. Cette température de fusion est une température de changement d'état du premier ordre.

Cette température de fusion peut être mesurée par toute méthode connue et en particulier à l'aide d'un calorimètre à balayage différentiel (D.S.C).

De façon avantageuse, le ou les polymères semi-cristallins auxquels s'applique l'invention présentent une masse moléculaire moyenne en nombre supérieure ou égale à 1 000. De façon avantageuse, le ou les polymères semi-cristallins mis en oeuvre selon l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000. Par « chaîne ou séquence cristallisable », on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la « chaîne pendante cristallisable » peut être chaîne comportant au moins 6 atomes de carbone.

Le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

De tels polymères sont décrits par exemple dans le document EP 1396259. Selon un mode plus particulier de réalisation de l'invention, le polymère est issu d'un monomère à chaîne cristallisable choisi parmi les (méth)acrylates d'alkyle saturés en C₁₄ à C₂₂.

A titre d'exemple particulier de polymère semi-cristallin structurant utilisable selon l'invention, on peut citer les produits Intelimer^{®} de la société Landec décrits dans la brochure « Intelimer^{®} polymers », Landec IP22 (Rev. 4-97). Ces polymères sont sous forme solide à température ambiante (25 °C). Ils sont porteurs de chaînes latérales cristallisables.

### Gélifiants lipophiles

Les gélifiants utilisables selon l'invention peuvent être des gélifiants lipophiles organiques ou minéraux, polymériques ou moléculaires.

Comme gélifiant lipophile minéral, on peut citer les argiles éventuellement modifiées comme les hectorites modifiées par un chlorure d'ammonium en C₁₀ à C₂₂, comme l'hectorite modifiée par du chlorure de di-stéaryl di-méthyl ammonium telle que, par exemple, celle commercialisée sous la dénomination de Bentone 38V^{®} par la société ELEMENTIS.

On peut également citer la silice pyrogénée décrite précédemment dans texte. Les gélifiants lipophiles organiques polymériques sont par exemple les organopolysiloxanes élastomériques partiellement ou totalement réticulés, de structure tridimensionnelle, comme ceux commercialisés sous les dénominations de KSG6^{®}, KSG16^{®} et de KSG18^{®} par la société SHIN-ETSU, de Trefil E-505C^{®} et Trefil E-506C^{®} par la société DOW-CORNING, de Gransil SR-CYC^{®}, SR DMF10^{®}, SR-DC556^{®}, SR 5CYC gel^{®}, SR DMF 10 gel^{®} et de SR DC 556 gel^{®} par la société GRANT INDUSTRIES, de SF 1204^{®} et de JK 113^{®} par la société GENERAL ELECTRIC ; l'éthylcellulose comme celle vendue sous la dénomination Ethocel^{®} par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB^{®} par la société BASF, du type polystyrène/copoly(éthylène-propylène) tels que ceux commercialisés sous la dénomination de Kraton^{®} par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel^{®} comme par exemple le mélange de copolymère tribloc butylène/éthylène/styrène et de copolymère étoile éthylène/propylène/styrène dans l'isododécane (Versagel M 5960).

Comme gélifiant lipophile, on peut encore citer les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619; en particulier les résines de polyamides (notamment comprenant des groupes alkyles ayant de 12 à 22 atomes de carbone) telles que celles décrites dans US-A-5783657. A titre d'exemple de résine de polyamide pouvant être mise en oeuvre selon la présente invention, on peut citer UNICLEAR 100 VG^{®} commercialisé par la société ARIZONA CHEMICAL.

Parmi les gélifiants lipophiles pouvant être utilisés selon l'invention, on peut encore citer les esters de dextrine et d'acide gras, tels que les palmitates de dextrine, notamment tels que ceux commercialisés sous les dénominations Rheopearl TL^{®} ou Rheopearl KL^{®} par la société CHIBA FLOUR.

On peut également utiliser les polyamides siliconés du type polyorganosiloxane tels que ceux décrits dans les documents US-A-5,874,069, US-A-5,919,441, US-A-6,051,216 et US-A-5,981,680.

Ces polymères siliconés peuvent appartenir aux deux familles suivantes :
- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

La gaine ou phase solide peut, en outre, comprendre d'autres corps gras tels que les huiles volatiles ou non volatiles citées précédemment pour autant que leurs teneurs et/ou leur nature n'affectent pas les propriétés de dureté de la gaine solide.

Selon un mode de réalisation préféré, le rapport pondéral gaine solide/coeur liquide varie de 0,1 à 5, et de préférence de 0,5 à 3.

Avantageusement, dans une composition selon l'invention, la gaine solide a une épaisseur représentant de 10 % à 90 % du rayon du stick, plus particulièrement de 20 % à 80 %, voire de 25 % à 75 % du rayon du stick.

Outre leur phase grasse respective, le coeur liquide comme la gaine solide (l'ensemble étant défini ci-après comme « composition », sauf indication contraire), peuvent comprendre les composants additionnels usuellement utilisés en cosmétique, tels que des matières colorantes, des charges ou des actifs cosmétiques.

Ces composants additionnels sont présents en des quantités ajustées de manière à ce que les propriétés d'une composition conforme à l'invention ne soient pas substantiellement altérées, notamment en terme de dureté, viscosité et/ou brillance.

### Matière colorante

Une composition conforme à la présente invention peut comprendre au moins une matière colorante pouvant être choisie parmi les matières colorantes hydrosolubles ou non, liposolubles ou non, organiques ou inorganiques, notamment de type pigments ou nacres, classiquement utilisée dans les compositions cosmétiques, les matériaux à effet optique, et leurs mélanges.

Les matières colorantes peuvent être présentes à raison de 0,01 à 40 % en poids, de préférence de 0,5 à 25 % en poids par rapport au poids total de la composition.

Par pigments, il faut comprendre des particules blanches ou colorées, inorganiques (minérales) ou organiques, insolubles dans une solution aqueuse, destinées à colorer le film résultant.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence « COVERLEAF NS » ou « JS » par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence « PC BALL PC-LL-100 P », ce pigment étant constitué de microsphères de silice contenant de l'oxyde de fer jaune.

On peut également, de préférence, mettre en oeuvre dans des compositions selon l'invention, du borosilicate de calcium et sodium sous forme de plaquette de borosilicate de calcium et sodium enrobées de titane et de dioxyde d'étain (94,25/5,25/0,5).

Parmi les pigments organiques utilisables dans l'invention, on peut citer le noir de carbone, les pigments de type D & C, les laques à base de carmin de cochenille, de baryum, strontium, calcium, aluminium ou encore les dicétopyrrolopyrroles (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica titane recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres « TIMICA », « FLAMENCO » et « DUOCHROME » (sur base de mica) commercialisées par la société ENGELHARD, les nacres « TIMIRON » commercialisées par la société MERCK, les nacres sur base de mica « PRESTIGE » commercialisées par la société ECKART et les nacres sur base de mica synthétique « SUNSHINE » commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté par exemple commercialisées par la société MERCK sous la dénomination Xirona Silver et les nacres orangées rosées vert doré notamment commercialisées par la société MERCK sous la dénomination Indian summer (Xirona) et leurs mélanges.

La composition cosmétique selon l'invention peut également contenir au moins un matériau à effet optique spécifique.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles.

Les particules à reflet métallique utilisables dans l'invention sont en particulier choisies parmi :
- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

A titre illustratif de ces particules, on peut citer des particules d'aluminium, telles que celles commercialisées sous les dénominations « STARBRITE 1200 EAC^{®} » par la société SIBERLINE et « METALURE^{®} » par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations « ROTOSAFE 700 » de la société ECKART, les particules d'aluminium enrobé de silice commercialisées sous la dénomination « VISIONAIRE BRIGHT SILVER » de la société ECKART et les particules d'alliage métallique comme des poudres de bronze (alliage cuivre et zinc) enrobé de silice commercialisées sous la dénomination de « VISIONAIRE BRIGHT NATURAL GOLD » de la société ECKART.

Il peut encore s'agir de particules comportant un substrat de verre comme celles commercialisées par la société NIPPON SHEET GLASS sous les dénominations « MICROGLASS METASHINE ».

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination « CHROMAFLAIR » par la société FLEX ; MOS₂/SiO₂/Al/SiO₂/MOS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination « SICOPEARL » par la société BASF ; MoS₂/SiO₂/mica-oxyde/SiO₂/MOS₂ , Fe₂O₃/SiO₂/mica-oxyde/SiO₂/Fe₂O₃, TiO₂/SiO₂/TiO₂ et TiO₂/Al₂O₃/TiO₂ ; SnO/TiO₂/SiO₂/TiO₂/SnO, Fe₂O₃/SiO₂/Fe₂O₃ ; SnO/mica/TiO₂/SiO₂/TiO₂/mica/SnO, des pigments ayant ces structures étant commercialisés sous la dénomination « XIRONA » par la société MERCK (Darmstadt). A titre d'exemple, ces pigments peuvent être les pigments de structure silice/oxyde de titane/oxyde d'étain commercialisés sous le nom « XIRONA MAGIC » par la société MERCK, les pigments de structure silice/oxyde de fer brun commercialisés sous le nom « XIRONA INDIAN SUMMER » par la société MERCK et les pigments de structure silice/oxyde de titane/mica/oxyde d'étain commercialisés sous le nom « XIRONA CARRIBEAN BLUE » par la société MERCK. On peut encore citer les pigments « INFINITE COLORS » de la société SHISEIDO. Selon l'épaisseur et la nature des différentes couches, on obtient différents effets. Ainsi, avec la structure Fe₂O₃/SiO₂/Al/SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination « COLOR GLITTER ».

Comme particules goniochromatiques à cristaux liquides, on peut utiliser par exemple celles vendues par la société CHENIX ainsi que celle commercialisées sous la dénomination « HELICONE^{®} HC » par la société WACKER.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave, le bleu de méthylène.

### Charges

Les compositions cosmétiques conformes à l'invention peuvent également comprendre au moins une charge, de nature organique ou minérale.

Par « charge », il faut comprendre les particules incolores ou blanches, solides de toutes formes, qui se présentent sous une forme insoluble et dispersée dans le milieu de la composition. De nature minérale ou organique, elles permettent de conférer du corps ou de la rigidité à la composition, et/ou de la douceur, de la matité et de l'uniformité au maquillage. Elles sont distinctes des particules de silice pyrogénée et des matières colorantes

Parmi les charges minérales utilisables dans les compositions selon l'invention, on peut citer le talc, le mica, la silice, le siloxysilicate de triméthyle, le kaolin, la bentone, le carbonate de calcium précipité, le carbonate et l'hydrogéno-carbonate de magnésium, l'hydroxyapatite, le nitrure de bore, les microsphères de silice creuses (Silice Beads de Maprecos), les microcapsules de verre ou de céramique ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass, et leurs mélanges.

Il peut notamment s'agir de charges sphériques comme par exemple le talc, le stéarate de zinc, le mica, le kaolin, les poudres de polyamide (Nylon^{®}) (Orgasol^{®} de chez Atochem), les poudres de polyéthylène, les poudres de polymères de tétrafluoroéthylène (Téflon^{®}), l'amidon, le nitrure de bore, des microsphères polymériques telles que celles de chlorure de polyvinylidène/acrylonitrile comme l'Expancel^{®} (Nobel Industrie), de copolymères d'acide acrylique (Polytrap^{®} de la société Dow Corning) et les microbilles de résine de silicone (Tospearls^{®} de Toshiba, par exemple), les organopolysiloxanes élastomères.

Parmi les charges organiques utilisables dans les compositions selon l'invention on peut citer les poudres de polyamide (Nylon^{®} Orgasol de chez Atochem), de poly-b-alanine et polyéthylène, les poudres de polytétrafluoroéthylène (Téflon®), la lauroyl-lysine, l'amidon, les poudres de polymères de tétrafluoroéthylène, les microsphères creuses de polymères telles l'EXPANCEL (NOBEL INDUSTRIE), le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le Polypore^{®} L 200 (Chemdal Corporation), les microbilles de résine de silicone (Tospearl^{®} de Toshiba, par exemple), les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400^{®} ou PLASTIC POWDER D-800^{®} de la société TOSHIKI, et leurs mélanges.

Une charge peut être présente dans le coeur liquide selon l'invention dans une teneur variant de 0,1 à 10 %, de préférence de 0,1 à 5 % et de préférence de 0.5 à 3 % en par rapport au poids du coeur liquide.

Une charge peut être présente dans la gaine solide selon l'invention dans une teneur variant de 0.1 à 20 %, de préférence de 0.5 à 15 % et de préférence de 1 à 5 % en par rapport au poids de la gaine solide.

Avantageusement, la totalité des charges est présente dans la gaine solide. En particulier, la gaine solide contient au moins deux fois plus de charge que le coeur liquide.

### Ingrédients cosmétiques usuels additionnels

La composition selon l'invention peut comprendre en outre tout ingrédient cosmétique usuel pouvant être choisi notamment parmi les antioxydants, les parfums, les conservateurs, les neutralisants, les tensioactifs, les filtres solaires, les édulcorants, les vitamines, les hydratants, les émollients, les actifs hydrophiles ou lipophiles, les agents anti-radicaux libres, les sequestrants, et leurs mélanges.

Bien entendu, l'homme du métier veillera à choisir les éventuels ingrédients complémentaires et/ou leur quantité de telle manière que les propriétés avantageuses de la composition selon l'invention ne soient pas ou substantiellement pas altérées par l'adjonction envisagée.

### Procédé de préparation

Le coeur liquide et la gaine solide peuvent être préparés selon les procédés connus, généralement utilisés dans le domaine cosmétique ou dermatologique.

De façon générale, la composition destinée à former la gaine solide est coulée à chaud à l'intérieur d'un moule comprenant un noyau central amovible au diamètre souhaité pour la partie liquide. Après refroidissement, le noyau central du moule est retiré, via l'extrémité du moule opposée à celle définissant la surface d'application, généralement biseautée du stick, laissant ainsi place à un tube dans lequel une canule est introduite. La composition, après chauffage éventuel en vue d'en abaisser la viscosité, est alors introduite au moyen de la canule dans le tube jusqu'à remplissage de ce dernier. Après refroidissement, le stick est sorti du moule, et conditionné ensuite de manière conventionnelle.

L'invention est illustrée plus en détail dans les exemples suivants donnés à titre illustratif et sans caractère limitatif. Les pourcentages sont des pourcentages en poids.

### Exemple 1 : Préparation d'une formule liquide du coeur

La formule liquide du coeur est réalisée conformément aux procédés bien connus de l'homme du métier.

On prépare un produit de maquillage de type « gloss » pour les lèvres dont la composition est la suivante :

| Ingrédient | % en poids du coeur liquide |
|---|---|
| Trimellitate de tridécyle (Liponate TDM^{®} de LIPO CHEMICALS) | 34,07 |
| Polyisobutène hydrogéné (PARLEAM^{®} de NOF) | 34,07 |
| Polybutène (INDOPOL H-100^{®}) | 20 |
| Condensat diacide en C₃₆ hydrogéné/éthylène diamine, estérifié par alcool stéarylique (poids moléculaire d'environ 4 000) stabilisé (Uniclear 100 VG^{®} de ARIZONA CHEMICAL) | 0,4 |
| Silice pyrogénée hydrophobe, traitée en surface par di-méthylsilane (AEROSIL R 972^{®} de DEGUSSA) | 10,5 |
| Conservateurs | qs |
| Parfum | 0,5 |
| Total | 100 |

Le mode opératoire utilisé est le suivant.
Le polybutène, le polyisobutylène, le tri-méllitate de tri-décyle, le polyamide et les conservateurs, sont mélangés à température de fusion du polyamide, soit environ 105 °C.

Une fois le mélange homogène et le polyamide bien fondu, on ajoute progressivement le parfum, et les particules de silice pyrogénée en maintenant la température à 100-105 °C au Rayneri.

Le mélange est agité jusqu'à homogénéisation complète et obtention d'un mélange translucide.

Pour plus de transparence, on peut soumettre ce mélange au Rayneri sous-vide pour éliminer les éventuelles bulles d'air présentes dans le produit fini.

La composition est ensuite coulée dans la gaine solide.

### Exemple 2 : Préparation d'une formule liquide du coeur

On mélange du tri-méllitate de tri-décyle avec de l'isoparaffine (6 à 8 moles d'isobutylène), un copolymère dilinoléyle diol/dilinoléiques et le polybutène (monooléfme/isoparaffine - 85/15) à une température de 90 °C. On ajoute ensuite à ce mélange des corps gras additionnels tels que des cires, plus précisément, une cire de polyéthylène à 100 °C. On mélange le tout jusqu'à homogénéisation puis on disperse la silice et la nacre au Rayneri.

La composition de la formule ainsi obtenue est détaillée ci-dessous.

| **Ingrédient** | **% en poids du coeur liquide** |
|---|---|
| Ditertiobutyl 4-hydroxytoluène | 0,06 |
| Silice pyrogénée hydrophobe,traitée en surface par di-méthylsilane | 4 |
| Plaquette de borosilicate de calcium et sodium enrobée de dioxyde de titane et de dioxyde d'étain (94.25/5.25/0.5) | 3 |
| Trimellitate de tridécyle (Liponate TDM^{®} de LIPO CHEMICALS) | 24,94 |
| Parfum | 0,5 |
| Isoparaffine (6-8 moles d'isobutylène) hydrogénée | 26 |
| Polybutène (Monooléfines / Isoparaffines (85/15)) (PM : 1290) | 10 |
| Cire de polyéthylène (PM: 500) | 1.5 |
| Copolymère dimères dilinoléyle diol/ dimères dilinoléiques | 30 |
| TOTAL | 100 |

### Exemple 3 : Préparation de la formule solide constituant la gaine solide

La composition de l'écorce solide est conforme à celle d'un bâton de rouge à lèvres traditionnel, ce dernier étant particulièrement choisi pour ses qualités d'application et de confort.

| Phase A | % en poids de la gaine solide |
|---|---|
| Hydroxytoluène butylé (BHT) | 0,06 |
| Copolymère de PEG-45/Glycol de dodécyle (Elfacos ST9^{®} de AKZO NOBEL) | 6 |
| Néopentanoate d'octyldodécyle | 18 |
| Polybutène | 15 |
| Triisostéarine | 7 |
| Octyldodécyle/PPG-3 Myristyle éther dilinoléate dimère | 1 |
| Polyacyladipate-2 de bis-diglycéryle (Softisan 649^{®} de SASOL) | 15 |
| Isostéarate d'isostéaryle | 10 |
| Phase B | |
| Distéardimonium hectorite (Bentone 38V^{®} de ELEMENTIS) | 1 |
| Phase C | |
| Yellow 6 Lake^{®} | 7 |
| Red 7^{®} | 4 |
| Dioxyde de titanium | 1 |
| Phase D | |
| Cire de polyéthylène (Performalène 500-L polyéthylène^{®} de NEW PHASE TECHNOLOGIES) | 5 |
| Cire microcristalline (Microwax HW^{®} de PARAMELT) | 7 |
| Phase E | |
| Mica | 2,94 |

Dans un poêlon on ajoute les ingrédients de la phase A. On agite le tout avec un Rayneri en chauffant légèrement à 50 °C.

On broie les pigments de la phase C dans une partie de la phase A.

On disperse la Bentone 38V^{®} (Phase B) dans l'autre partie de la phase A.

Dans un poêlon double paroi on ajoute le broyat, l'hectorite dispersée dans la phase A et les cires de la phase D. On chauffe le tout à 98-100 °C en agitant au Rayneri jusqu'à fusion des cires.

Enfin, la phase E est ajoutée au mélange, que l'on coule dans un moule pour réaliser des sticks de diamètre 12.7 mm.

### Exemple 4 : Composition selon l'invention comprenant un coeur liquide et une écorce solide

On peut réaliser des compositions cosmétiques selon l'invention en mettant en oeuvre les compositions préparées selon les exemples 1, 2 et 3. Pour ce faire, on utilise un moule double enveloppe dans lequel la composition solide destinée à former l'écorce est coulée en premier dans l'enveloppe du moule la plus externe. Dans une deuxième étape, la formulation destinée à former le coeur liquide (exemple 1 ou 2), de la composition est coulée dans l'enveloppe interne de l'écorce précédente.

## Revendications

1. Composition cosmétique, notamment sous forme de stick, formée d'au moins un coeur liquide comprenant au moins une phase grasse liquide à température ambiante, et retenu à l'intérieur d'une gaine solide comprenant au moins un corps gras solide à température ambiante.

2. Composition selon la revendication précédente dans laquelle le coeur liquide est retenu à l'intérieur de la gaine solide par capillarité.

3. Composition selon l'une quelconque des revendications précédentes, dans laquelle le coeur liquide comprend au moins une huile non volatile, choisie parmi les polybutènes, les polyisobutylènes hydrogénés, les polydécènes, les polydécènes hydrogénés, les huiles de silicone, l'oligomère vinylpyrrolidone/1-hexadécène, l'isononanoate d'isonoyle, les esters de polyol(s) et de dimère diacide d'acide gras ou un de ses esters, les esters aromatiques tels que le tridécyle trimellitate, et leurs mélanges.

4. Composition selon l'une quelconque des revendications précédentes, dans laquelle le coeur liquide comprend au moins un ester de polyol(s) et de dimère diacide d'acide gras, ou un de ses esters.

5. Composition selon l'une quelconque des revendications précédentes, dans laquelle le coeur liquide contient en outre des particules de silice pyrogénée.

6. Composition selon la revendication précédente, dans laquelle les particules de silice pyrogénée sont présentes en une teneur variant de 1 % à 30 % en poids, en particulier de 2 à 20 % en poids, et plus particulièrement de 5 à 15 % en poids par rapport au poids total du coeur liquide.

7. Composition selon l'une quelconque des revendications précédentes, dans laquelle la gaine solide comprend au moins une cire.

8. Composition selon la revendication précédente dans laquelle la cire est présente dans une teneur d'au moins 1 % en poids par rapport au poids de la gaine solide et plus particulièrement dans une teneur variant de 3 à 40 %, de préférence de 5 à 30 %, tout particulièrement de 5% à 25% en poids, voire de 5 à 20 % en poids par rapport au poids de la gaine solide.

9. Composition selon l'une quelconque des revendications précédentes dans laquelle la gaine solide a une dureté comprise entre 30 et 500 g, en particulier entre 100 et 350 g, voire entre 100 et 300 g.

10. Composition selon l'une quelconque de revendications précédentes dans laquelle la viscosité à 25 °C du coeur liquide varie de 50 à 400 Poises et de préférence de 80 à 250 Poises.

11. Composition selon l'une quelconque des revendications précédentes, dans laquelle le rapport pondéral gaine solide/coeur liquide varie de 0,1 à 5, et de préférence de 0,5 à 3.

12. Composition selon l'une quelconque des revendications précédentes, **caractérisé en ce qu'**elle comprend en outre au moins une matière colorante présente en une teneur variant de 0,01 à 40 %, de préférence de 0,5 à 25 %, en poids par rapport au poids total de la composition.

13. Composition selon la revendication précédente, **caractérisé en ce que** ladite matière colorante est choisie parmi les matières colorantes hydrosolubles ou non, liposolubles ou non, organiques ou inorganiques, notamment de type pigments ou nacres, les matériaux à effet optique, et leurs mélanges.

14. Composition selon l'une quelconque des revendications précédentes, ladite composition étant anhydre.

15. Procédé cosmétique de soin et/ou de maquillage des matières kératiniques, consistant à appliquer sur ces dernières, au moyen d'un stick, une composition telle que définie selon l'une des revendications 1 à 14.

16. Procédé cosmétique dans lequel une composition telle que définie selon l'une des revendications 1 à 14 est sous la forme d'un stick.
